# EUROPEAN PATENT SPECIFICATION

(11) **EP 3 342 379 B1**
(45) Date of publication and mention of the grant of the patent: **23.10.2019**
(21) Application number: 16306821.6
(22) Date of filing: 27.12.2016
(51) Int. Cl.: A61F 7/10, A61F 7/02, A61F 7/00

(54) **NON-SHIVERING CRYOTHERMOGENESIS METHOD FOR REDUCING ADIPOSE TISSUES**
VERFAHREN ZUR ZITTERFREIEN KRYOTHERMOGENESE ZUR REDUZIERUNG VON ADIPÖSEN GEWEBEN
PROCÉDÉ DE RÉDUCTION DES TISSUS ADIPEUX PAR CRYOTHERMOGÉNÈSE INDOLORE

(43) Date of publication of application: 04.07.2018
(73) Proprietor: Loap, Suvaddhana Sarin, 75007 Paris (FR)
(72) Inventor: Loap, Suvaddhana Sarin, 75007 Paris (FR)
(74) Representative: Hirsch & Associés

(56) References cited:
- EP-A1- 2 856 986
- US-A1- 2008 077 202
- US-A1- 2011 190 856
- US-B2- 7 367 341

## Description

The present invention relates to a non-therapeutic and non-invasive cosmetic method activating non-shivering thermogenesis to reduce adipose tissue without lipid-rich cells disruption in a subject expecting slimming subject by controlled cooling.

Excess body fat and localized adiposity represent important social problems and many subjects affected by it seek plastic surgery to improve their appearance. However, plastic surgery is an invasive medical procedure with associated risks, which generally may allow a rapid recovery and may affect the social life.

Indeed, even with regular physical exercises, it is difficult to eliminate fat from lipid-rich cells or adipose tissue, as they usually only burn their fats in extreme conditions, such as diet. However, diets bring only short-term benefits and present the disadvantage to be long, difficult, requiring to change the entire lifestyle and, lead in many cases to regain lost weight. Accordingly, various cosmetic procedures and devices have been developed to remove or reduce unwanted local subcutaneous fat.

Attention has focused on alternative methods for adipose tissue (AT) disruption including radiofrequency, ultrasound and laser treatment. In recent years, body cooling or "cryolipolysis" has been developed, it involves either environmental cold exposure (Bartelt et al.) or devices to reduce skin surface temperature to -7°C (e.g., U.S. Patent No. 9,308,120 and U.S. Patent No.7,367,341, Example 1), leading to systemic or local exposure of fatty tissues to active cooling. Cryolipolysis development was based on studies showing that intracellular lipid content varies among cell types, with dermal and epidermal cells being relatively low in unsaturated fatty acids compared to the underlying adipocytes that form the subcutaneous fatty tissue. As a result, the different cell types, e.g. lipid-rich and non-lipid-rich cells, exhibit varying degrees of susceptibility to the cold, with non-lipid-rich cells withstanding colder temperatures than lipid-rich cells. Therefore, cryolipolysis is using these properties by applying cooling means to crystallize cytoplasmic lipids, disrupt cellular integrity and induce cell death via apoptosis/necrosis, to selectively reduce AT over a period of weeks to months (U.S. Patent No. 9,308,120).

Accordingly, due to the adipocyte cell death, methods disrupting cellular integrity as cryolipolysis present the inconvenient of:
- localized bruising, frostbite, swelling or dulling of sensation may occur (which clears within a few weeks);
- developing hard lump due to the crystallization of cytoplasmic lipids, requiring massaging the lump after treatment;
- risks of paradoxical adipose hyperplasia (Singh et al. (2015), and Jalian et al. (2014)), which can require surgery in some severe cases;
- generating fat dead cells debris, fat (lipotoxicity), cytokines and other inflammatory mediators that the organism has to gradually digest and eliminate in the weeks or months after the procedure, which restrain the treatment to a limited number of local regions at the same time.

These inconvenient lead to widely spaced treatment (e.g., several days or weeks apart) to allow the subject to eliminate said cell debris and fat, to resolve inflammation and other side effects.
However, lipid-rich cells or adipose tissue are very useful as they are involved in numerous metabolism processes, such as storing excess energy as lipid, regulating systemic energy balance through the release of adipokines that target peripheral tissues (Dempersmier et al., 2015), targeting the brain to modulate appetite in response to excess energy supply (Sonna et al., 1985), and protecting tissues including muscle and liver from lipotoxicity (Haemmerle et al., 2006). Accordingly, convenient and efficient non-therapeutic or cosmetic methods to improve subject appearance by reducing adipose tissue without causing injury to lipid rich cells (e.g. adipose tissue) were heretofore unknown. In this context, the applicant surprisingly found a method using controlled-cooling to boost energy expenditure via fat metabolism and non-shivering thermogenesis, which does not present the inconvenient related to lipid rich cell disruption described above. Accordingly, the method of the invention was shown to promote reduction of adipose tissue without lipid rich cell disruption, reducing waist circumference with a single session of multiple applications, or to cumulate reduction of waist circumference with repeated sessions (even daily repeated sessions) with aesthetic purpose.
Others advantages of the present method over prior art methods, is that it does not promote inflammatory reaction or loose skin, and it increases venous return and it is active on lipomastia.

The present invention relates to a non-therapeutic non-shivering cryothermogenesis method for locally reducing adipose tissue of a subject's body without lipid-rich cell disruption, the non-shivering cryothermogenesis being defined as a cold-induced increase in heat production not associated with a muscle activity of shivering, said method, comprising:
(a) applying at least one applicator of a cooling mean to subject's skin to regions wherein subcutaneous adipose tissue is white adipose tissue (WAT) or contains beigeing or browning adipocytes which refers to white adipocytes transformation into brown adipocytes by cold; and
(b) applying further applicators of said cooling mean to local region wherein adipose tissue reduction is desired; and
(c) said applicators of said cooling mean maintaining skin superficial temperature between 4°C and 12°C, preferentially between 6°C and 10°C, more preferentially about 8°C; and
(d) applying said applicators for a total period of time comprised between 20 and 60 minutes, preferentially between 30 and 50 minutes, more preferentially about 40 minutes.

Advantageously, the method according to the invention further comprise in said step b) applying additional applicators of said cooling mean along the large vascular axes of the circulatory system, such as femoral artery, tibial artery, subclavian artery, axillary artery, brachial artery... taking care of not compressing them.
The term "cryothermogenesis" refers herein to a biological process activated by cold which induce or enhance thermogenesis, without causing cell death. By "thermogenesis" it is meant a process occurring in all warm-blooded animals, and consisting in cell producing heat from fatty acids and carbohydrates. During these metabolic process non-toxic end-products are released (e.g. H₂O and CO₂) and no inflammatory reaction is induced.
The terms "non-shivering cryothermogenesis" are defined as a cold-induced increase in heat production not associated with the muscle activity of shivering.

Accordingly, as shown herein no significant difference in the level of inflammatory biomarker(s) can be observed before, during and after the application of the method according to the invention.

By "reducing adipose tissue" it is meant herein that adipose tissue volume of at least one body part of a subject is reduced. This reduction in adipose tissue can be determined by measuring the thickness of the skin with an adipometer or by measuring circumference of the treated body part of the subject.

The term "inflammatory biomarker(s)" as used herein relates to biomarker(s) indicative of the presence of an inflammatory state, e.g. neopterin, sedimentation rate, C-reactive protein (CRP), and additional cell markers and cytokines identifiable by a skilled person.

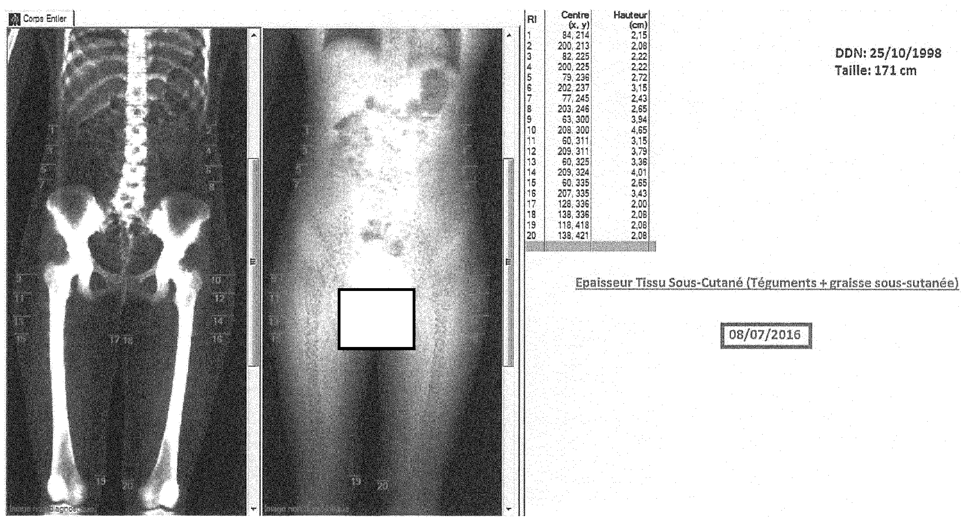

The term "subcutaneous adipose tissue" means tissue lying underneath the dermis and includes adipocytes (fat cells) and subcutaneous fat. By "subcutaneous adipocytes tissue rich in brown adipocytes" refers to areas localized along the spine (cervical, paravertebral), supraclavicular, axillary, pericardial, pericardial/renal/adrenal, trachea-esophageal, mediastinal, intercostal and mesenteric (Figure 1). The expression "white adipose tissue (WAT) containing brown adipocytes" or "brite adipocytes" (i.e. brown in white) or beige adipocytes refer to all areas of white adipose tissue that contains brown adipocytes. Beigeing or browning adipocytes refers to white adipocytes transformation into brown adipocytes by various factors, including cold.

The term "brown adipocyte" relates to cells present in BAT that are specialized in heat production (thermogenesis) in mammals. Two principal types of adipose tissue have been described, WAT and BAT. The most abundant adipose cell types are the white adipocytes that contain a single intracellular lipid droplet and localize to specific depots within the body. In contrast to WAT, BAT adipocytes express uncoupling proteins that permit mitochondria to metabolize fat via β-oxidation to generate heat (Avram et Harry, 2009). Uncoupling proteins 1, when activated, short circuits the electrochemical gradient that drives ATP synthesis and thereby stimulates respiratory chain activity. Heat is generated from the combustion of available substrates and is distributed to the rest of the body through the circulation. Similarly, "brown-like' adipocytes" or "beige fat cells" where also identified in adipose tissue and refers to cells containing significant amounts of Uncoupling proteins 1, providing them thermogenic capacities http://www.nature.com/nm/journal/v19/n10/full/nm.3361.html - ref10.

By "local region wherein adipose tissue reduction is desired" it is meant a defined region or area comprising adipocytes, and where cooling means will be applied to specifically reduce the adipose tissue present in this area, or to specifically activate thermogenesis through brown adipocytes and/or WAT containing brown adipocytes. According to a preferred embodiment, said local region are selected from the stomach, love handles, inner thighs, inner knees, butt, inner arms, chin, breast area, back and neck.

Therefore, the method according to the invention by reducing adipose tissue in a subject can reduce circumference of the waist, hips, upper thigh, lower thigh, calf, forearms, biceps, chest and/or shoulder, preferentially the circumference of the waist, hips and/or upper thigh and chin.

By "maintaining skin superficial temperature" it is meant herein that the cooling mean is adapted to cool the surface of the skin to a specific temperature and to maintain it by adapting the applied temperature. However, it will be obvious for a skilled person to adapt the applied temperature according to the duration of application, and conversely, to adapt the duration of application according to the applied temperature.
Said skin superficial temperature can be determined by any mean allowing to measure temperature, which are well known by one skilled in the art. For examples such mean allowing to measure temperature can be selected from infrared thermometer or laser thermometers or non-contact thermometers. Therefore, by maintaining skin superficial temperature at the temperature according to the invention, adipocyte cells are preserved from freezing-induced cell death, and any disruption of adipocyte cells is prevented.

By "about 8°C" it is meant a skin superficial temperature of 8°C ±2°C, preferentially 8°C ±1 °C. By "about 40 minutes" it is meant a period of time of 40 minutes ±4 minutes, preferentially 40 minutes ±2 minutes. Obviously, the skilled person performing the claimed method would be capable to adjust parameters in order to maintain the skin superficial temperature to about 8°C.
By "cooling mean" it is referred herein to any mean capable to cool and/or apply to a body part of a subject a specific set up temperature during a certain period of time, i.e. by removing heat or applying cold. The term "applicator" refers to a of time, i.e. by removing heat or applying cold. The term "applicator" refers to a part of a cooling mean that can be applied directly to a subject's skin and from which generated cold air is expelled to cool the skin surface of the subject. Of course, it would be obvious to the skilled person to adapt the shape or the size of said applicator to the local area to be cooled. Preferably, said applicators are able to apply negative pressure to form a sealed chamber in contact to the skin of a subject, wherein the specific set up temperature is applied in said chamber. Also, according to local region to be treated by the method according to the invention, the said applicators would be chosen according to the surface of the skin to be covered. Such cooling devices and applicators are commercially available and are for example supplied by Beijing ADSS Development Co., Ltd, or Beijing Jontelaser Technology Co., Ltd, or Beijing Himalaya Medical Technology Co., Ltd.

In a particular embodiment, to perform the method according to the invention, applicators of said cooling mean are applied to said subject's skin in a number comprised at least 2, preferentially between 5 to 14, more preferentially between 8 to 12. Preferentially, said applicator of said cooling mean cover a surface area comprised between 60 and 90 cm², preferentially between 70 and 80 cm². Advantageously said applicator has a rectangular shape. It will be obvious for a skilled person to adapt the shape and/or size of said applicators to said local region wherein adipose tissue reduction is desired.

Moreover, it will be obvious for a skilled person to adapt the number and/or the shape and/or the size of applicators to the subject's build in order to cover the largest surface area of the subject's skin.

In a preferred embodiment, said applicators of said cooling mean cover between 30% and 70%, preferentially between 40% and 55% of the back or the front of the subject upper body (i.e. from the neck to the lower back or pelvis).

In a preferred embodiment, said applicators of said cooling mean cover between 60 cm² and 120 cm², preferentially between 70 cm² and 100 cm² of the subject body.

In a preferred embodiment, said applicators cover between 19% and 33.5%, preferentially between 22.5% and 30% of the back of the subject.

In a preferred embodiment, said applicators cover between 14% and 31.5%, preferentially between 17.5% and 25% of the front of the subject.

In a preferred embodiment, the temperature applied by said applicator in the method according to the invention is comprised between -15°C to 5°C, preferentially -10°C to 0°C, more preferentially -9°C to -3°C.

In a particular embodiment, time periods of step d) of the method according to the invention is divided in two or three subperiods, preferentially three subperiods, said subperiods during between 8 and 25 minutes each, preferentially during 10 or 20 minutes each.

In a preferred embodiment, the period of time of step d) of the method according to the invention is comprised between 25 and 75 minutes, between 30 and 65 minutes, preferentially between 35 and 55 minutes, more preferentially about 40 minutes.

In a preferred embodiment, the method according to the invention comprises three subperiods consisting in:
i. about 10 minutes at about -10°C; and
ii. about 10 minutes at about -8°C; and
iii. about 20 minutes at about -7°C.

In another embodiment, said applicators of said cooling mean according to the invention are applied to the subject's skin with a suction pressure comprised between 20 and 300 kPa, preferentially between 100 kPa and 200 kPa. Preferentially, said pressure is comprised between 20 kPa and 100 kPa when the thickness of the skin thickness of a subject is inferior or equal to about 3 cm, and is comprised between 101 kPa and 300 kPa when the thickness of the skin of a subject is superior to about 3 cm. The term "about 3 cm" meant that the thickness of the skin is 3 cm ±0.3, preferentially 3 cm ±0.2. Preferentially, said pressure should vary between 20 kPa to 100 kPa when said applicators of said cooling mean are applied along the large vascular axes of the circulatory system, such as femoral artery, tibial artery, subclavian artery, axillary artery, brachial artery...

The expression "the thickness of the skin" refers herein to the measurement of the thickness of the skin by an adipometer. It is known from the skilled person that adipometer measurements are used as a guide for calculating the amount of subcutaneous fat. However, it would be obvious for a skilled person to adapt threshold values with measurements obtained by any other means.

In a preferred embodiment, when said applicators are of rectangular shape, the method according to the invention comprised an additional step following said steps a) and/or b), wherein said applicators of rectangular shape are moved by 90 degrees and applied for an additional period of time. Advantageously, said additional step following said steps a) and/or b) is applied when the thickness of the skin of said subject is superior to about 3 cm.

Another object of the present invention is a cryothermogenesis method for reducing adipose tissue of a subject's body, comprising the step of:
(a) firstly applying the method according to the invention to the back of the subject from the neck to the lower back or pelvis; and then
(b) secondly applying the method according to the invention to the front of the subject from the neck to the lower back or pelvis.

Another object of the present invention is a cryothermogenesis method for locally reducing adipose tissue of subject's body, comprising repeating the method according to the invention to the subject's skin a plurality of times until the desired reduction in adipose tissue at a local region has been achieved, said reduction in adipose tissue being determined by measuring the thickness of the skin at the local region with an adipometer or by measuring circumference of the treated body part of the subject, wherein said repeating application can be applied as soon as said adipose tissue recover their temperature of prior said step (a) or (b). Preferentially, said plurality of times are spaced of about 60 minutes time intervals, or 1 hour time intervals, 12 hour time intervals, or 1 day time intervals, preferentially 1 day time intervals.
**Figure 1****.** Percentage change in (A) waist circumference, and (B) thigh circumference, following single abdominal applications of tissue cryotherapy (n = 17; treatment duration ca 1.33 h; time following treatment, ≤1 h). Error bars indicate SD.
**Figure 2****.** Individual responses to tissue cryotherapy as a function of age in subjects receiving single applications of tissue cryotherapy (trendline added, P = 0.2).
**Figure 3****.** Decrement in (A) waist circumference, (B) weight, and (C) BMI following three sequential daily tissue cryotherapy treatments (n = 12). Error bars indicate SD.

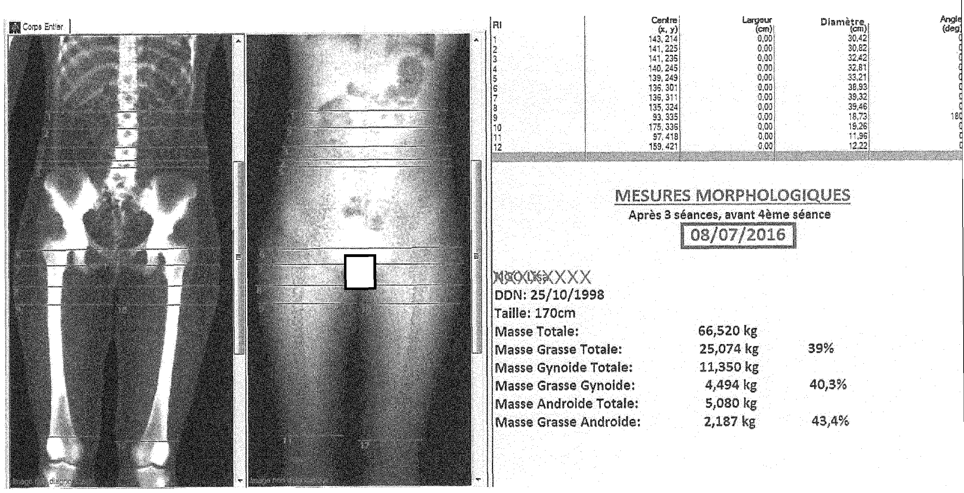

**Figure 4****.** Progressive decline in waist circumference (expressed as %) in five subjects undergoing repeat tissue cryotherapy treatments over four (subject 1, M, 59 years of age), five (subjects 2, 3, 4; F, 47 years; F, 71 years; F, 52 years), or seven (subject 5, M, 51 years) days. The mean decline after 5 treatments was 6.1%.

### EXAMPLES

### EXAMPLE 1- Effect of the cryothermogenesis procedure on waist circumference

### Methods

### 1. Subjects and ethical permissions

In this retrospective study data for 28 participants (9M/19F), mean age 51.5 (range 33-71 years) were analyzed. All had a BMI consistent with overweight. Thermogenesis method, a non-invasive procedure, does not require formal institutional ethical approval.

### 2. Tissue cryotherapy procedure

Treatment employed a commercial tissue cryotherapy device (FG6601-006, ADSS, Republic of China) equipped with multiple cooling probes (cooling area 20 × 8 cm). Weight, height, and waist and thigh (left) circumferences were recorded; BMI was calculated automatically using a commercial apparatus (model SC240MA, Tanita, Japan). A layer of wetted paper (glycerol-based wetting membrane, ETG-111-200, Freezefats, Republic of China) was applied to the lower back of the reclining subject, followed by symmetrical application of four to six pairwise probes accompanied by gentle suction to improve contact. Cooling temperature was set to -10°C, declining to -7°C over 20 minutes; application duration was 40 minutes. Subjects then lay supine and the treatment was repeated on the lower abdomen, again for 40 minutes. Oral temperature, a proxy for core temperature, was recorded before and after the procedure. Local skin surface temperatures were recorded immediately after removal of the cooling probe and measurement showed that it was maintained around +8°C and did not fall below +4°C. Weight, waist and thigh circumference, body weight, and BMI were recorded again after the procedure. 12 subjects volunteered for triple daily applications. Five participants (3 F and 2 M; aged 47, 51, 71; and 51, 57 years, respectively) volunteered for more extended daily applications, and the procedure was repeated on a daily basis for a total of four, five, or seven applications, with systematic recording as before.

### 3. Statistics

Statistical analyses employed normalized means and SD. Results were evaluated using the Student *t* test; statistical significance was set at *P*≤ 0.05.

### 4. Local cooling produces decrements in waist circumference

Waist circumference was recorded before and after single applications of local abdominal tissue cooling (cryothermogenesis method) in a series of 17 subjects. As shown in Fig. 1A, there was a mean reduction of 3.0% of waist circumference following the procedure. The decline was of high statistical significance (*P* = <0.0001). By contrast, there was no change in thigh circumference (Fig. 1B). Core body temperature (oral) rose from mean 35.8 to mean 36.1 (+0.3°C), but the change was not significant. Changes in weight and BMI with single applications fell short of statistical significance (not presented; see below).

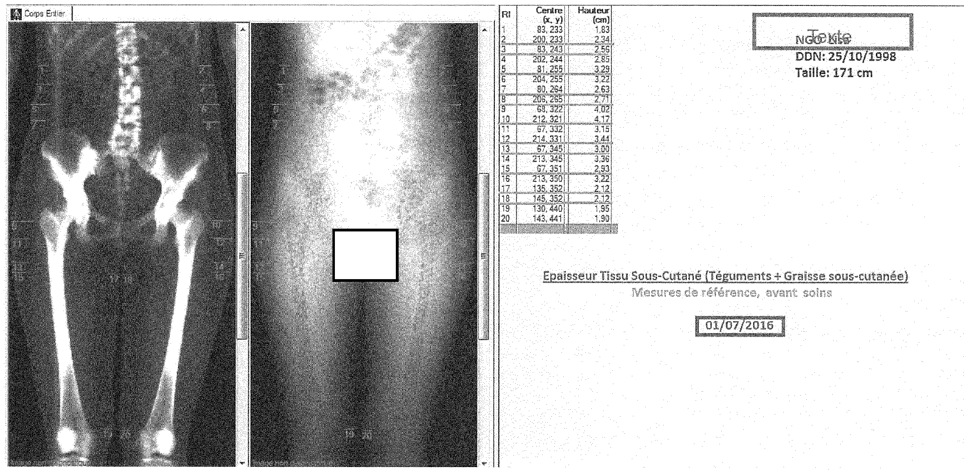

As shown in Fig. 2, there was a trend towards a reduction in efficacy as a function of age. However, the trend was not of statistical significance (P = -0.2): some older individuals displayed substantial reductions in waist circumference and some young subjects displayed low responses. There were no significant gender differences.

### 5. Multiple applications produce loss of waist circumference

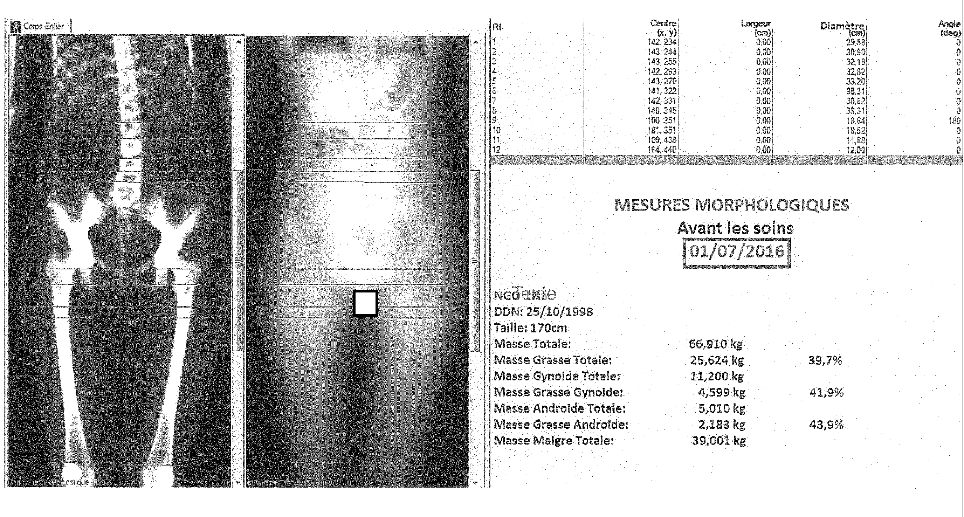

Twelve subjects undertook subsequent treatments on days 2 and 3 under the same conditions and for the same duration as on day 1. As shown in Fig. 3, in addition to a significant decline in waist circumference (*P* = 0.0068), highly significant reductions in both body weight (*P* = <0.001) and BMI (*P* = 0.013) were observed. The extent of mean reduction in waist circumference, 4.2%, exceeded the mean reduction obtained by single applications (3.0%, *P*= NS versus single application).

To address a possible delay-/time-dependent effect, nine subjects receiving a single treatment underwent further measurements 5 to 10 days later but without further tissue cryotherapy. Given the small number of subjects, the extent of the reduction in waist circumference (3.9%, SD = 3.09) did not achieve statistical significance versus the reduction observed immediately following single treatment (3.0%, SD = 2.71), although there was trend towards an increase.

Five subjects (3 F, 2 M) volunteered for repeat daily treatments over periods of 4, 5, and 7 days. As shown in Fig. 3, repeat treatment was accompanied, in all five subjects, by a progressive loss of waist circumference (mean loss of 6.1% immediately following five treatments), to be compared against a 3% mean loss immediately following a single treatment. The difference in waist loss following four to seven treatments was significantly greater than from single treatments (P = <0.01).

### 6. Biomedical parameters

Local skin temperatures immediately beneath the cooling probe were variable within the region, but did not fall below +6°C. There was some local reddening of the skin consistent with increased local blood circulation. No superficial skin damage of any type was observed. Comprehensive blood analysis was carried out on four subjects before and 3 days after treatment. Profiles before and after treatment were unremarkable. Importantly, except as noted, there was no indication of inflammation, macrophage activation, or abnormal lipid profiles. Therefore the cryothermogenesis method is not associated with systemic inflammation or changes in blood lipid profiles. In addition, no participant reported any adverse events.

### 7. Conclusion

Single applications of local abdominal tissue cooling (cryothermogenesis method) were found to produce a significant tissue reduction. All subjects responded to single treatments by a reduction in waist circumference (mean ∼3%) that was statistically significant (P = <0.0001). There was no evident M/F gender difference in response. There was an apparent trend towards to reduced efficacy with age; however, the trend was not statistically significant, and participants among the oldest in the group displayed responses to single treatments.

Results show further progressive loss of AT with multiple applications. A larger reduction in circumference was observed following three treatments (∼4.2%), and this was accompanied by decrements in both body weight (∼0.75%) and BMI (∼0.8%) that were statistically significant. The reduction in BMI indicates selective loss of AT volume. The reduction in waist circumference after three treatments (∼0.4%) was consistent with the decline in total body weight (∼0.75%).

In five subjects receiving extended rounds of treatment (four to seven), there was evidence for progressive decrements in waist circumference (∼6% after five treatments), arguing that multiple treatments at daily intervals produce cumulative benefits. No consistent changes in biochemical parameters or adverse events were reported for the participants in this study. These results confirm that local cooling of abdominal tissue produces selective loss of AT volume.

In contrast to earlier reports that there is no evident change in body fat immediately after treatment, the results showed significant beneficial effects within 1h of treatment, a time-course too rapid to be explained by cell death and clearance. In addition, multiple daily treatments produced progressive losses of waist circumference, bodyweight, and BMI which is incompatible with the cell disruption. Furthermore, biochemical analysis revealed no evidence of markers of inflammation or macrophage activation in treated subjects, showing adipocyte cell volume reduction via thermogenesis, with conservation of cellular integrity. In support, complete maintenance of core body temperature (+0.3°C) was recorded despite intense local cooling, attesting that thermogenesis is taking place. Results therefore point to energy expenditure via cold-induced fat metabolism and thermogenesis, with consequent reduction in adipocyte cell volume and AT mass.

Regarding the safety of the method according to the invention, no consistent changes in biochemical parameters were observed following treatment, including markers of inflammation. No adverse effects were reported by the participants.

### Examples 2 -Inflammatory Biomarkers measurements

### Method

### 1. Biomedical parameters

Comprehensive blood analysis was carried out on subjects before and 3 days after the daily treatment applied daily during 5 to 10 days. All profiles before and after treatment (±4 days) were unremarkable. Importantly, there was no indication of inflammation or macrophage activation.

### Results

**Table 1 - Measure of Neopterine before and after cryothermogenesis treatment**

| | | Neopterine (nmol/l) |
|---|---|---|
| Subject 1 | before | 3,3 |
| | after | 8 |
| Subject 2 | before | 2,9 |
| | after | 5,2 |
| Subject 3 | before | 9 |
| | after | 6,5 |
| Subject 4 | before | 4 |
| | after | 6,6 |
| Subject 5 | before | 8,1 |
| | after | 7,4 |

**Table 2 - Measure of the sedimentation rate (first hour and second hour) and C-reactive protein (CRP) before and after cryothermogenesis treatment**

| | | Sedimentation rate | CRP |
|---|---|---|---|
| Subject a | before | 7 and 19 | <5 |
| | after | 6 and 13 | <1 |
| Subject b | before | 4 and 9 | 3 |
| | after | 9 and 26 | 5 |
| Subject c | before | 3 and 6 | <5 |
| | after | 3 and 6 | <5 |
| Subject d | before | 6 and 14 | <5 |
| | after | 6 and 15 | 2 |
| Subject e | before | 2 and 6 | 5 |
| | after | 3 and 5 | <1 |

### Conclusions

Results of table 1 and 2 showed that cryothermogenesis method is not associated with systemic inflammation as neopterine (normal value: <25 nmol/l, the sedimentation rates (normal values: first hour 1 to 20 and second hour 4 to 40) and CRP (normal value: <6 mg/L), which are well known by the skilled person, are in the range of their respective normal values. In addition, no participant reported any adverse events.

### References

M.M. Avram, R.S. Harry, Cryolipolysis for subcutaneous fat layer reduction. Lasers Surg.Med. 41 (2009) 703-708.
A. Bartelt, O.T. Bruns, R. Reimer, H. Hohenberg, H. Ittrich, K. Peldschus, M.G. Kaul, U.I. Tromsdorf, H. Weller, C. Waurisch, A. Eychmuller, P.L. Gordts, F. Rinninger, K. Bruegelmann, B. Freund, P. Nielsen, M. Merkel, J. Heeren, Brown adipose tissue activity controls triglyceride clearance. Nat.Med. 17 (2011) 200-205.
J. Dempersmier, A. Sambeat, O. Gulyaeva, S.M. Paul, C.S. Hudak, H.F. Raposo, H.Y. Kwan, C. Kang, R.H. Wong, H.S. Sul, Cold-inducible Zfp516 activates UCP1 transcription to promote browning of white fat and development of brown fat. Mol.Cell. 57 (2015) 235-246.
G. Haemmerle, A. Lass, R. Zimmermann, G. Gorkiewicz, C. Meyer, J. Rozman, G. Heldmaier, R. Maier, C. Theussl, S. Eder, D. Kratky, E.F. Wagner, M. Klingenspor, G. Hoefler, R. Zechner, Defective lipolysis and altered energy metabolism in mice lacking adipose triglyceride lipase. Science. 312 (2006) 734-737.
HR Jalian; MM Avram; L Garibyan; MC Mihm; R.R Anderson. Paradoxical Adipose Hyperplasia After Cryolipolysis. JAMA Dermatol. 2014;150(3):317-319.
SM Singh, ER Geddes, SG Boutrous, RD Galiano, PM Friedman. Paradoxical adipose hyperplasia secondary to cryolipolysis: An underreported entity? Lasers Surg Med. (2015); 47(6):476-8.
L.A. Sonna, J. Fujita, S.L. Gaffin, C.M. Lilly, Invited review: Effects of heat and cold stress on mammalian gene expression. J.Appl.Physiol.(1985.) 92 (2002) 1725-1742.

## Claims

1. Anon-therapeutic non-shivering cry other mogenesis method for locally reducing adipose tissue of a subject's body without lipid-rich cell disruption, the non-shivering cryothermogenesis being defined as a cold-induced increase in heat production not associated with a muscle activity of shivering, said method comprising:
(a) applying at least one applicator of a cooling means to the subj ect's skin to a region wherein subcutaneous adipocytes tissue is white adipose tissue (WAT) or contains beigeing or browning adipocytes which refers to white adipocytes transformation into brown adipocytes by cold ; and
(b) applying at least one additional applicator of said cooling means to a local region wherein adipose tissue reduction is desired, if said local region differs from the one defined in step (a);
(c) said applicator of said cooling means maintaining skin superficial temperature between 4°C and 12°C, preferentially between 6°C and 10°C, more preferentially about 8°C; and
(d) applying said applicators for a total period of time comprised between 20 and 60 min, preferentially between 30 and 50 min, more preferentially about 40 minutes.

2. The method of claim 1, wherein said applicator applies a temperature comprised between -15°C to 5°C, preferentially -12°C to 0°C, more preferentially -9°C to -3°C.

3. The method of claim 1 or 2, wherein said time periods of step d) is divided in two or three subperiods, preferentially three subperiods, each of said subperiod during between 10 and 20 minutes.

4. The method of claim 3, wherein said three subperiods consist in:
i. applying a temperature of -10°C during 10 minutes; and
ii. applying a temperature of -8°C during 10 minutes; and
iii. applying a temperature of -7°C during 20 minutes.

5. The method of any one of claims 1 to 4, wherein said local region is preferentially selected from the stomach, love handles, inner thighs, inner knees, butt, inner arms, chin, breast area, back and neck.

6. The method of any one of claims 1 to 4, wherein at least 2 applicators are applied to said subject's skin, preferentially 5 to 14, more preferentially 8 to 12.

7. A cryothermogenesis method for reducing adipose tissue of a subject's body, comprising the step of:
(a) firstly applying the method of any one of claims 1 to 6 to the back of the subject from the neck to the lower back or pelvis; and then
(b) secondly applying the method of any one of claims 1 to 6 to the front of the subject from the neck to the lower back or pelvis.

8. A cryothermogenesis method for locally reducing adipose tissue of subject's body, comprising repeating the method according to any of claims 1 to 6 to the subject's skin a plurality of times until the desired reduction in adipose tissue at a local region has been achieved, said reduction in adipose tissue being determined by measuring the thickness of the skin at the local region with an adipometer or by measuring circumference of the treated body part of the subject, wherein said application can be repeated as soon as said adipose tissue recover their temperature of prior said step (a) or (b).

9. The method of claim 7, wherein said plurality of times are spaced of 1 hour time intervals, 12 hour time intervals or 1 day time interval, preferentially 1 day time interval.

10. The method of any one of claims 1 to 4 and 6, wherein said applicators of said cooling mean cover between 30%and 70% preferentially between 40%and 55% of the back or the front of the subject upper body.

11. The method of any one of claims 1 to 4 and 6, wherein said applicators of said cooling mean cover between 19%and 33.5% preferentially between 22.5%and 30%of the back of the subject.

12. The method of any one of claims 1 to 4 and 6, wherein said applicators of said cooling mean cover between 14%and 31.5% preferentially between 17.5%and 25%of the front of the subject.

## Patentansprüche

1. Nicht-therapeutisches zitterfreies Kryothermogenese-Verfahren zur lokalen Reduktion des Fettgewebes des Körpers einer Person ohne lipidreiche Zellstörung, wobei die zitterfreie Kryothermogenese als eine kälteinduzierte Erhöhung der Wärmeproduktion definiert wird, die nicht im Zusammenhang mit einer Muskelaktivität des Zitterns steht, wobei dieses Verfahren umfasst:
a) Anwendung von mindestens einem Applikator eines Kühlmittels auf die Haut der Person in einer Region, in der subkutanes Adipozytengewebe weißes Fettgewebe (WAT) ist oder beige werdende oder bräunende Adipozyten enthält, was sich auf die Umwandlung von weißen Adipozyten in braune Adipozyten durch Kälte bezieht; und
b) Anwendung von mindestens einem zusätzlichen Applikator dieses Kühlmittels auf eine lokale Region, in der eine Reduktion des Fettgewebes gewünscht wird, wenn diese lokale Region sich von der in Schritt (a) definierten unterscheidet;
c) der Applikator dieses Kühlmittels, der die oberflächliche Hauttemperatur zwischen 4 °C und 12 °C, bevorzugt zwischen 6 °C und 10 °C, am besten bei 8 °C hält; und
d) Anwendung der Applikatoren über einen Gesamtzeitraum von 20 bis 60 min, bevorzugt zwischen 30 und 50 min, am besten etwa 40 Minuten.

2. Verfahren nach Anspruch 1, bei dem der Applikator eine Temperatur zwischen -15 °C bis 5 °C, bevorzugt -12 °C bis 0 °C, am besten von -9 °C bis -3 °C anwendet.

3. Verfahren nach Anspruch 1 oder 2, wobei die genannten Zeiträume von Schritt d) in zwei oder drei Teilperioden, vorzugsweise drei Teilperioden, jeweils in diesem Teilzeitraum zwischen 10 und 20 Minuten unterteilt sind.

4. Verfahren nach Anspruch 3, wobei die drei Teilperioden in folgenden bestehen:
I. Anwenden einer Temperatur von -10 °C über 10 Minuten; und
II. Anwenden einer Temperatur von -8 °C über 10 Minuten; und
III. Anwenden einer Temperatur von -7 °C über 20 Minuten.

5. Verfahren nach einem der Ansprüche 1 bis 4, wobei die lokale Region bevorzugt aus dem Magen, Rettungsringen, innerem Oberschenkel, innerem Knie, Hintern, inneren Armen, Kinn, Brustbereich, Rücken und Hals ausgewählt wird.

6. Verfahren nach einem der Ansprüche 1 bis 4, wobei mindestens 2 Applikatoren auf die Haut dieser Person aufgebracht werden, bevorzugt 5 bis 14, am besten 8 bis 12.

7. Kryothermogenese-Verfahren zur Verringerung des Fettgewebes des Körpers einer Person, die folgendes umfasst:
a) erstens das Anwenden des Verfahrens nach einem der Ansprüche 1 bis 6 auf die Rückseite der Person vom Hals bis zum unteren Rücken oder Becken; und dann
b) zweitens Anwenden des Verfahrens nach einem der Ansprüche 1 bis 6 auf die Vorderseite der Person vom Hals bis zum unteren Rücken oder Becken anwenden.

8. Kryothermogenese-Verfahren zur lokalen Reduktion des Fettgewebes des Körpers des Betreffenden, die die Wiederholung des Verfahrens nach einem der Ansprüche 1 bis 6 auf die Haut des Betreffenden mehrere Male umfasst, bis die gewünschte Reduktion des Fettgewebes in einer lokalen Region erreicht worden ist, wobei die Reduzierung des Fettgewebes durch Messung der Dicke der Haut in der lokalen Region mit einem Adipometer oder durch Messung des Umfangs des behandelten Körperteils der Person bestimmt wird, wobei diese Anwendung wiederholt werden kann, sobald dieses Fettgewebe seine Temperatur vom vorherigen Schritt (a) oder (b) wiedererlangt.

9. Verfahren nach Anspruch 7, wobei die Vielzahl der Zeiten im Abstand von 1 Stunde Zeitintervall, 12 Stunden Zeitintervall oder 1 Tag Zeitintervall, vorzugsweise 1 Tag Zeitintervall verteilt ist.

10. Verfahren nach einem der Ansprüche 1 bis 4 und 6, wobei die Applikatoren dieses Kühlmittels zwischen 30 % und 70 %, bevorzugt zwischen 40 % und 55 % der Rückseite oder der Vorderseite des Oberkörpers der Person abdecken.

11. Verfahren nach einem der Ansprüche 1 bis 4 und 6, wobei die Applikatoren dieses Kühlmittels zwischen 19 % und 33,5 % abdecken, bevorzugt zwischen 22,5 % und 30 % der Rückseite des Betreffenden.

12. Verfahren nach einem der Ansprüche 1 bis 4 und 6, wobei die Applikatoren dieses Kühlmittels zwischen 14 % und 31,5 %, bevorzugt zwischen 17,5 % und 25 % der Vorderseite des Betreffenden abdecken.

## Revendications

1. Procédé de cryothermogenèse sans frisson, non-thérapeutique, pour réduire localement le tissu adipeux du corps d'un sujet sans rupture des cellules riches en lipides, la cryothermogenèse sans frisson étant définie par une augmentation induite par le froid de la production de chaleur non associée à une activité musculaire de frisson, ledit procédé comprenant :
(a) l'application d'au moins un applicateur d'un moyen de refroidissement sur la peau d'un sujet sur une région dans laquelle le tissu adipeux sous-cutané est du tissu adipeux blanc (WAT) ou contient des adipocytes devenant beiges ou devenant marron, ceci se référant à la transformation d'adipocytes blancs en adipocytes marron par le froid ; et
(b) l'application d'au moins un applicateur additionnel dudit moyen de refroidissement sur une région locale dans laquelle une réduction du tissu adipeux est souhaitée, si ladite région locale diffère de celle définie dans l'étape (a) ;
(c) ledit applicateur dudit moyen de refroidissement maintenant la température superficielle de la peau entre 4°C et 12°C, de préférence entre 6°C et 10°C, plus préférentiellement à environ 8°C ; et
(d) l'application desdits applicateurs pour une période de temps totale comprise entre 20 et 60 minutes, de préférence entre 30 et 50 minutes, plus préférentiellement d'environ 40 minutes.

2. Procédé selon la revendication 1, dans lequel ledit applicateur applique une température comprise entre -15°C et 5°C, de préférence entre -12°C et 0°C, plus préférentiellement entre -9°C et -3°C.

3. Procédé selon la revendication 1 ou 2, dans lequel ladite période de temps de l'étape d) est divisée en deux ou trois sous-périodes, de préférence trois sous-périodes, chacune desdites sous-périodes durant entre 10 et 20 minutes.

4. Procédé selon la revendication 3, dans lequel lesdites trois sous-périodes consistent en :
i. l'application d'une température de -10°C pendant 10 minutes ; et
ii. l'application d'une température de -8°C pendant 10 minutes ; et
iii. l'application d'une température de -7°C pendant 20 minutes.

5. Procédé selon l'une quelconque des revendications 1 à 4, dans lequel ladite région locale est de préférence choisie parmi l'estomac, les poignées d'amour, l'intérieur des cuisses, l'intérieur des genoux, les fesses, l'intérieur des bras, le menton, la région de la poitrine, le dos et le cou.

6. Procédé selon l'une quelconque des revendications 1 à 4, dans lequel au moins 2 applicateurs sont appliqués sur ladite peau du sujet, de préférence 5 à 14, mieux encore 8 à 12.

7. Procédé de cryothermogenèse pour réduire le tissu adipeux du corps d'un sujet, comprenant les étapes suivantes :
(a) en premier lieu application du procédé de l'une quelconque des revendications 1 à 6 sur le dos du sujet, du cou au bas du dos ou au pelvis ; et ensuite
(b) en second lieu application du procédé de l'une quelconque des revendications 1 à 6 sur l'avant du sujet, du cou au bas du dos ou au pelvis.

8. Procédé de cryothermogenèse pour réduire localement le tissu adipeux du corps d'un sujet, comprenant la répétition du procédé selon l'une quelconque des revendications 1 à 6 sur la peau du sujet plusieurs fois jusqu'à ce que la réduction souhaitée du tissu adipeux dans une région locale ait été obtenue, ladite réduction du tissu adipeux étant déterminée par mesure de l'épaisseur de la peau dans la région locale avec un adipomètre ou par mesure de la circonférence de la partie du corps traitée du sujet, dans lequel ladite application peut être répétée dès que ledit tissu adipeux reprend sa température d'avant l'étape (a) ou (b).

9. Procédé selon la revendication 7, dans lequel lesdites plusieurs fois sont espacées par des intervalles de temps de 1 heure, des intervalles de temps de 12 heures ou des intervalles de temps de 1 jour, de préférence des intervalles de temps de 1 jour.

10. Procédé selon l'une quelconque des revendications 1 à 4 et 6, dans lequel lesdits applicateurs dudit moyen de refroidissement couvrent entre 30 % et 70 %, de préférence entre 40 % et 55 % du dos ou de l'avant du corps supérieur du sujet.

11. Procédé selon l'une quelconque des revendications 1 à 4 et 6, dans lequel lesdits applicateurs dudit moyen de refroidissement couvrent entre 19 % et 33,5 %, de préférence entre 22,5 et 30 % du dos du sujet.

12. Procédé selon l'une quelconque des revendications 1 à 4 et 6, dans lequel lesdits applicateurs dudit moyen de refroidissement couvrent entre 14 % et 31,5 %, de préférence entre 17,5 et 25 % de l'avant du sujet.
